# EUROPEAN PATENT APPLICATION

(11) **EP 3 831 927 A1**
(43) Date of publication of application: **09.06.2021**
(21) Application number: 19848554.2
(22) Date of filing: 06.08.2019
(51) Int. Cl.: C12M 3/08

(54) **DEVICE FOR DIVIDING CELL MASS, AND METHOD FOR DIVIDING CELL MASS USING SAME**

(30) Priority: 06.08.2018 JP 2018148033
(71) Applicant: Nissan Chemical Corporation, Tokyo 103-6119 (JP)
(72) Inventor: OTSUKA, Keiichiro, Shiraoka-shi, Saitama 349-0294 (JP); MINAMI, Masataka, Funabashi-shi, Chiba 274-0052 (JP); HAYASHI, Hisato, Tokyo 103-6119 (JP)
(74) Representative: J A Kemp LLP
(86) International application number: PCT/JP2019/030940
(87) International publication number: WO 2020/032042

(57) **Abstract**

The device has a film-shaped main body part 1, and a predetermined region in the film surface of the main body part has a mesh structure in which a large number of through-holes 20 are arranged. The through-hole has an opening shape having a size allowing smaller cell aggregates to pass through, and the rest of the through-hole is the beam part 30. The beam part is a part that cuts a cell aggregate to be divided, and is integrally connected to form, a network. The cell aggregate can be divided by passing the cell aggregate to be divided through the mesh structure of the device together with the liquid.

## Description

### [Technical Field]

The present invention relates to a device for dividing cell aggregates, and a method for dividing cell aggregates by using the device.

### [Background Art]

In recent years, a cell culture method (also called a suspension culture method) has been developed in which various cells such as pluripotent stem cells and the like are suspended in a liquid medium and three-dimensionally grown into a cell aggregate (e.g., patent document 1 and the like). In addition, a liquid medium for preferably performing the suspension culture method and a production method thereof have also been developed (e.g., patent document 2 and the like).

In suspension culture method, the undifferentiated state of pluripotent stem cells may decrease as the cell aggregate grows larger. For example, non-patent document 1 suggests that the undifferentiated state of large cell aggregates of 150 µm or more may decrease.

On the other hand, in the culture method of pluripotent stem cells described in patent document 1, pluripotent stem cells are suspension cultured until they become large cell aggregates having an average diameter of about 200 - about 300 µm, the obtained large cell aggregates are divided into smaller cell aggregates having an average diameter of about 80 to about 120 µm, after which suspension culture is further continued to maintain and amplify the pluripotent stem cells. In this culture method, a mesh made by knitting nylon or metal wire is used as a specific method for dividing large cell aggregates, and large cell aggregates are passed through the mesh to be divided into small cell aggregates corresponding to the mesh-holes (square pass holes) of the mesh.

### [Document List]

### [Patent documents]

patent document 1: WO 2013/077423
patent document 2: WO 2016/163444

### [Non-patent document]

non-patent document 1: Andreas Elanzew et al., "A reproducible and versatile system for the dynamic expansion of human pluripotent stem cells in suspension", Biotechnology Journal, 2015, 10, 1589-1599.

### [Summary of Invention]

### [Technical Problem]

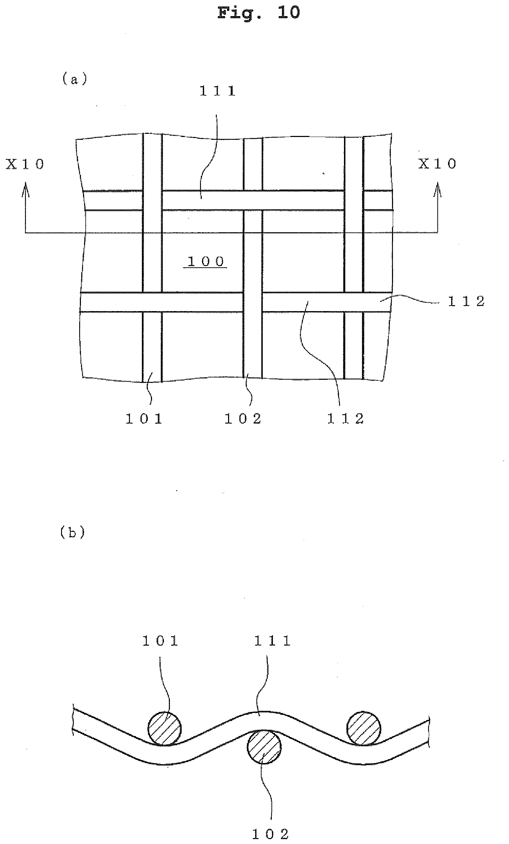

However, when the present inventors have examined in detail the division of the cell aggregates using the mesh as described above, it was found that the cell aggregates passing through the mesh may not be preferably divided due to the structure peculiar to the mesh. The mesh is a kind of sheet-like material, and when the sheet surface is seen macroscopically in a straight view, the warp wire and the weft wire appear to intersect linearly as shown in Fig. 10(a), and the mesh-hole also looks like a flat plane square. However, when each mesh-hole is microscopically observed, since the warp wire and the weft wire are knitted three-dimensionally so as to avoid each other, four wires (two warp wires (101, 102) and two weft wires (111, 112)) constituting four sides surrounding one square mesh-hole 100 wave broadly in the thickness direction of the mesh as shown in Fig. 10 (b) .

When cell aggregates pass through such mesh-hole surrounded by four wavy wires, since the cross-sectional shape of each wire is a circular shape, and the surface of the wire body is a curved surface, the cell aggregates may not be divided appropriately or sharply in some cases. When a thinner wire is used to improve such defect of the mesh, the strength of the mesh is reduced. When this is improved by increasing the wire strength, the mesh becomes more expensive. When cell aggregates are divided by a mesh formed using a wire and the flow velocity of a liquid medium is low, the cell aggregates cannot be cut but are only trapped in the mesh of the net. As a result, dividing and culture cannot be repeated and the collection rate of the cell aggregates becomes low. Therefore, a certain level of high flow velocity is necessary. On the other hand, when the flow velocity of a liquid medium is high, the divided cell aggregates receive a shear due to the high-speed flow and become smaller. It is not preferable to divide cell aggregates of pluripotent stem cells to have an outer diameter of 40 µm or less, since the cells are significantly damaged as evidenced by apoptosis of the cell and the like.

As described above, when cell aggregates are divided using a conventional mesh, a low flow velocity of a liquid medium leads to a low cell recovery rate and a high flow velocity of a liquid medium leads to large damage on the cell aggregates and low expansion culture efficiency.

The present invention aims to provide a device that can solve the above-mentioned problem and divide cell aggregates more preferably, and a method for dividing cell aggregates by using the device.

### [Solution to Problem]

The present inventors have conducted intensive studies in an attempt to solve the above-mentioned problems and found that a porous film having many through-holes disposed on the film surface to form mesh-holes, and a beam part having a sharp corner, compared with mesh wires, on the body surface is free from waving of the beam part surrounding the through-holes and can cut cell aggregates more preferably, which resulted in the completion of the present invention.

The main constitution of the present invention is as follows.
[1] A device for dividing a cell aggregate into smaller cell aggregates, the device comprising a film-like main body part, wherein
   a predetermined region on a film surface of the main body part has a mesh structure with many through-holes disposed on the film surface, the mesh structure comprises many through-holes penetrating the predetermined region in the film thickness direction, and a beam part serving as a partition between the through-holes,
   the through-holes have an opening shape of a size permitting passage of the aforementioned smaller cell aggregates,
   and the beam part is a remainder after subtracting the through-hole from the main body part in the predetermined region, is a part that cuts the cell aggregates to be divided, and is integrally connected to form a network.
[2] The device according to [1], wherein the opening shape of the through-hole has an opening area with an equivalent-circle-diameter of 40 µm - 90 µm, and a shape accommodating a circle with a diameter of 35 µm - 85 µm.
[3] The device according to [1] or [2], wherein the beam part has a width of 10 µm - 60 µm that is a separation distance between adjacent through-holes.
[4] The device according to any of [1] to [3], wherein said many through-holes have opening shapes of quadrangles congruent with each other, and said beam parts are connected to each other in an orthogonal lattice pattern.
[5] The device according to any of [1] to [3], wherein said many through-holes have opening shapes of hexagons congruent with each other, and said beam parts are connected to each other in a honeycomb-shape.
[6] The device according to [5], wherein the hexagon is a regular hexagon, and, among the six sides of the regular hexagon, a distance between two parallel sides facing each other is 38 µm - 85 µm.
[7] The device according to any of [1] to [6], wherein
   the aforementioned film surface is a first film surface, a film surface on the opposite side thereof is a second film surface,
   when in use of the device, the first film surface is a surface used as an inlet side, the second film surface is a surface used as an outlet side, and
   a cross-sectional shape in the perpendicular longitudinal direction of the aforementioned beam part is a rectangle, or two corners on the inlet side of the rectangle have a round shape.
[8] The device according to any of [1] to [7], wherein the cell aggregate to be divided is a cell aggregate composed of pluripotent stem cells.
[9] A method for dividing a cell aggregate, comprising a step of dividing a cell aggregate to be divided by passing, using the device of any of the above-mentioned [1] to [8], the cell aggregate together with a liquid through the mesh structure of the aforementioned device.
[10] The method according to [9], wherein the flow velocity of the liquid is 10 mm/sec - 500 mm/sec when the cell aggregate to be divided passes through the net-like region in the aforementioned device together with a liquid.
[11] The method according to [9] or [10], further comprising a backflow washing step of passing, after division of a predetermined amount of the cell aggregates in the aforementioned step of dividing the cell aggregate, a predetermined liquid through the mesh structure in the direction opposite to the direction of passage of the cell aggregate through the mesh structure of the device for division, thereby washing the mesh structure.

### [Advantageous Effects of Invention]

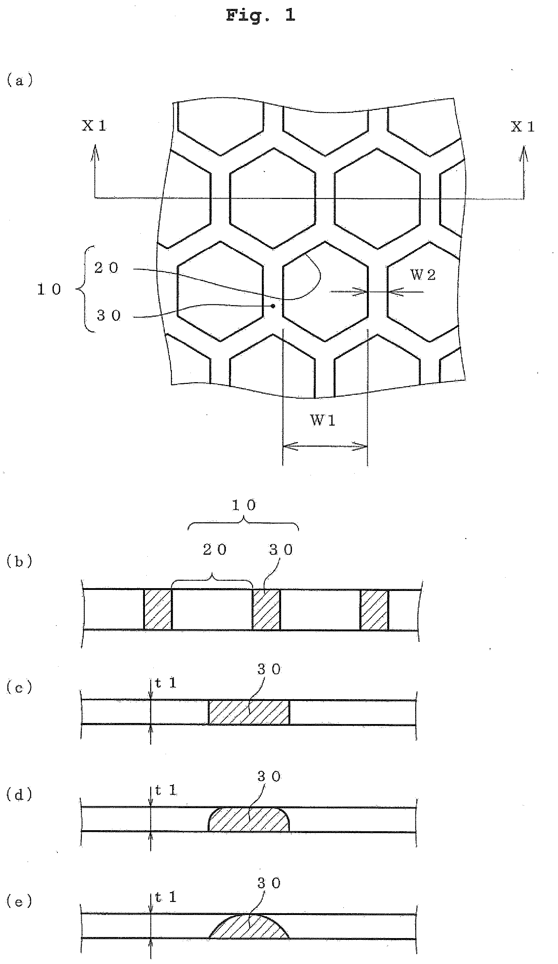

In the device of the present invention (hereinafter to be referred to as the device), a large number of through-holes are arranged on the film surface, and a predetermined region (a part or all of the region) of the film surface has a mesh structure. This mesh structure is a kind of porous film composed of through-holes that function as mesh-holes and beam parts that function as partition parts between adjacent through-holes. In the region of this mesh structure, as shown in Fig. 1(a), the beam part 20 is two-dimensionally connected as a net as the remainder of the film excluding the through-holes, and there is no waviness of mesh wires. Therefore, the cell aggregate that collides with such a beam part free of waving can be more preferably divided than the wavy mesh wires described above. Furthermore, as shown in Fig. 1(b) or Fig. 1(c), the cross-sectional shape of the beam part 20 is not a circle but close to a quadrangle or a rectangle (rectangle with long side in thickness direction, rectangle with short side in thickness direction, or square) . Therefore, the edge of the beam part 20 at the opening of each through-hole can divide the cell aggregate sharply without resistance, and thus the damage to the cell aggregate at the time of cutting may be smaller in some cases.

When dividing cell aggregates by using the device, therefore, the flow velocity of the liquid passing through the net-like region (such as a liquid medium in which the cell aggregates to be divided are dispersed) can be made lower than in the case of division using a conventional mesh, and crushing of the cell aggregate into excessively fine cell aggregates can also be suppressed.

In a preferred embodiment of the device, the opening shape of the through-hole is a shape closer to a circle (e.g., square or equilateral hexagon), and the width of the beam part is uniform. As a result, the damage to the cell aggregate at the time of cutting is smaller, and a sphere-shaped preferable cell aggregate having a uniform size can be obtained.

In the device, it is relatively easy in processing to narrow the width of the beam part. When the opening shape is a regular hexagon, the strength of the entire mesh structure is high, and the width of the beam part can be further narrowed. Since the cell aggregate can be further divided without resistance and the aperture ratio (the ratio of the opening to the total area of the mesh structure region) can be increased, the above-mentioned problem of mesh can be solved.

### [Brief Description of Drawings]

Fig. 1 shows an example of a preferred embodiment of the mesh structure in the device of the present invention. Fig. 1(a) is a partially enlarged view of a predetermined region of the film surface of the film-like main body part, and Fig. 1(b) is a cross-sectional view taken along the line Xl-Xl of Fig. 1(a). Fig. 1(c) - (e) show other embodiment of the cross section of the beam part shown in Fig. 1(b). In Fig. 1(b) -(e), the cross section of the beam part is hatched.

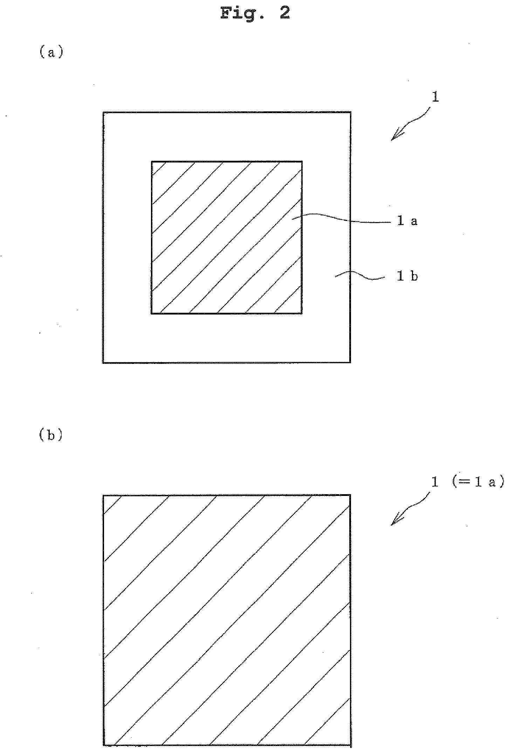

Fig. 2 illustrates the region where the mesh structure is provided on the film surface of the main body part of the device of the present invention. In this Figure, the opening of the through-hole is not shown, and the area where the mesh structure is provided is shown by hatching.

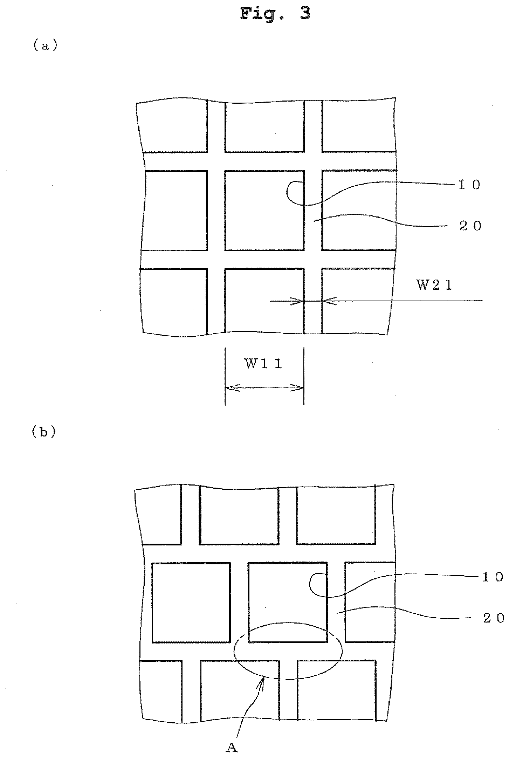

Fig. 3 shows other example of a preferred embodiment of the mesh structure in the device of the present invention, and is a partially enlarged view of a predetermined region of the film surface of the main body part.

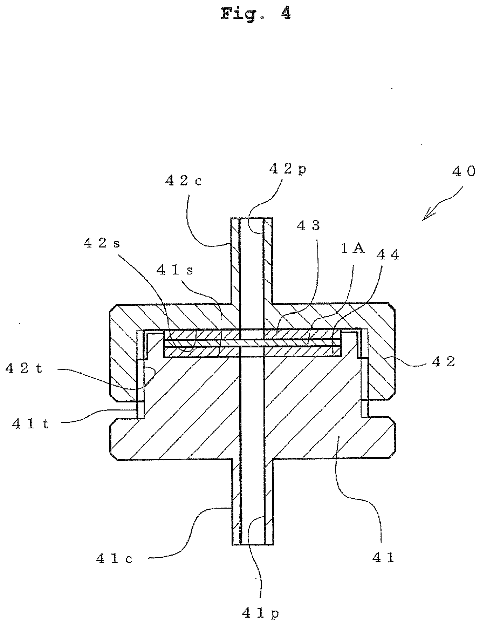

Fig. 4 is a cross-sectional view showing one embodiment of the structure of a device holder configured so that the device of the present invention can be preferably used for dividing a cell aggregate.

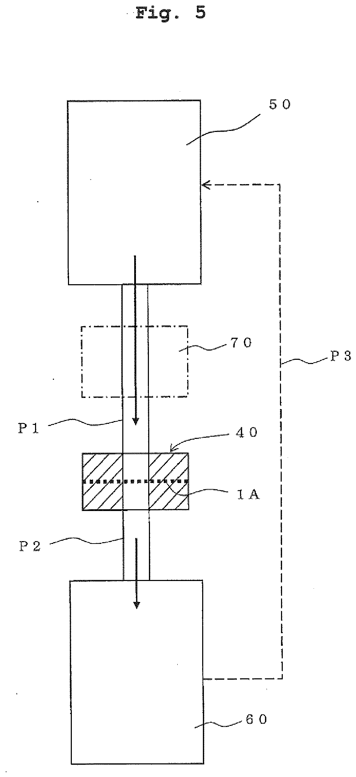

Fig. 5 schematically shows a method for dividing a cell aggregate according to the present invention. In the Figure, for the sake of explanation, the culture container is drawn small and the pipe line is drawn thick, but in reality, the culture container is of the size of a general culture bag, and the pipe line is a flexible piping tube.

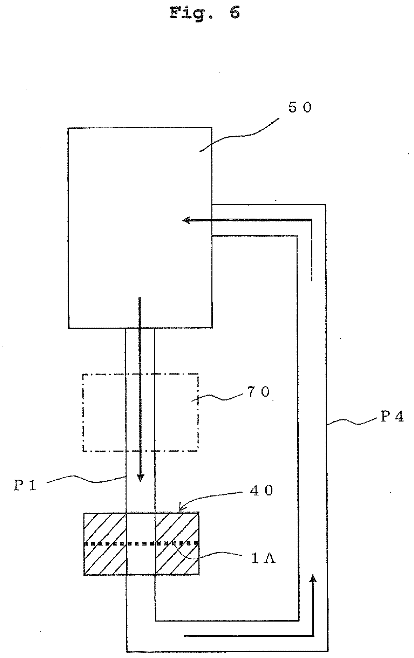

Fig. 6 schematically shows other embodiment of a method for dividing a cell aggregate according to the present invention. In the Figure, for the sake of explanation, the culture container is drawn small and the pipe line is drawn thick, but in reality, the culture container is of the size of a general culture bag, and the pipe line is a flexible piping tube.

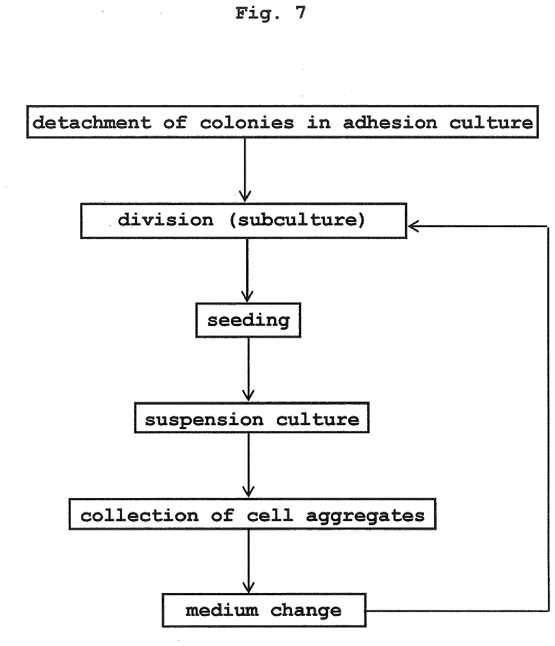

Fig. 7 is a flowchart showing one embodiment of a cell culture method using the method for dividing a cell aggregate according to the present invention.

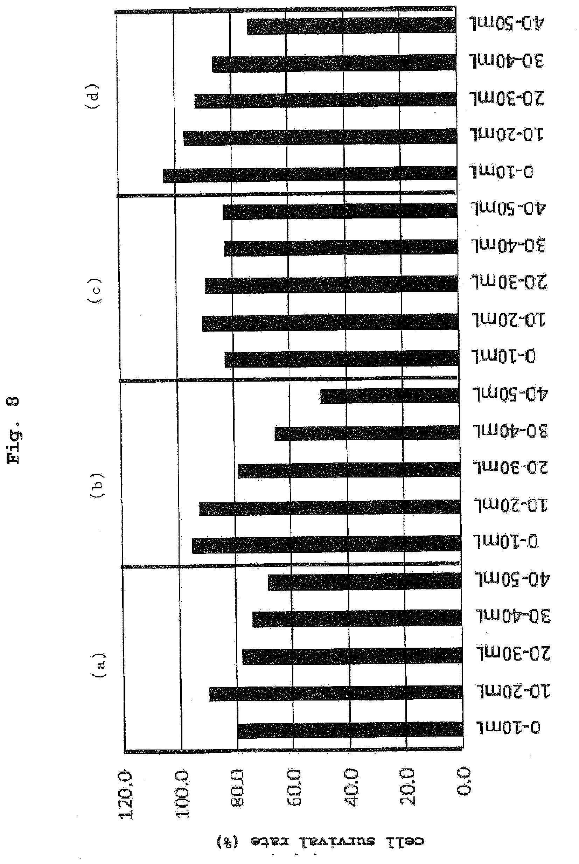

Fig. 8 is a graph showing the test results of examining the effect of backflow washing on the device according to the present invention. Fig. 8(a), (b) are graphs showing the survival rate of cells contained in the divided cell aggregates when the cell aggregate division is continued without performing backflow washing. Fig. 8(c), (d) are graphs showing the survival rate of cells contained in the divided cell aggregates when the cell aggregate division is continued while performing backflow washing when a predetermined amount is divided.

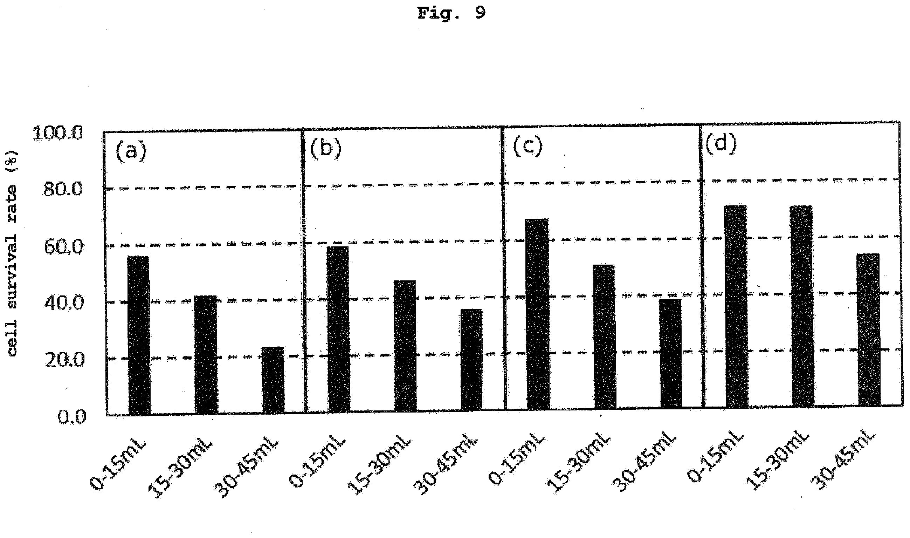

Fig. 9 is a graph showing the test results of investigation of the relationship between the cross-sectional shape of the beam part of a mesh structure, and the division performance in the present invention.
Fig. 10 shows the structure of a conventional mesh used for dividing cell aggregates. Fig. 10(a) is a partially enlarged view showing a predetermined region of the sheet surface of the mesh, and Fig. 10(b) is a cross-sectional view taken along the line X10-X10 of Fig. 10(a). In Fig. 10 (b), the cross section of the wire is hatched.

### [Description of Embodiments]

The device of the present invention is described in detail in the following with reference to Examples.

The device is used for dividing a cell aggregate that has grown big into smaller cell aggregates. The device has a film-like main body part. A highly rigid frame, tab, or the like may be further provided on the outer peripheral edge portion or the like of the main body part to improve handleability. In the embodiments described below, the entire device is a film-like main body part, and thus the entire device is a single film.

As shown in Fig. 1(a), a predetermined region on the film surface of the main body part of the device has a mesh structure 10 in which a large number of through-holes 20 are arranged on the film surface. The predetermined region on the film surface may be a part or all of the film surface. For example, in the case of Fig. 2(a), an outer circumference region 1b of a film-like main body part 1 is a flat film free of a through-hole, a through-hole is provided in the center region 1a. When the visible outline defining the outer circumference of the mesh structure (e.g., the boundary line of the outer circumference of the central region 1a) crosses the opening of the through-hole, the opening of the through-hole is a small opening only inside the visible outline. In this case, the through-hole of the small opening may be provided as it is, or the through-hole of such a small opening may not be provided, or a through-hole may be provided so that the opening with the original shape exceeds the visible outline.

### (Mesh structure)

As shown in Fig. 1(a), a mesh structure 10 is composed of a large number of through-holes 20 penetrating the aforementioned predetermined region in the direction of the film thickness, and a beam part 30 which is a partition between the through-holes. The through-hole 20 has an opening shape large enough to allow the aforementioned smaller cell aggregate (cell aggregate after division) to pass through. On the other hand, the beam part 30 is a remainder obtained by subtracting the aforementioned through-hole from the main body part in the aforementioned predetermined region. The beam part functions as a part for cutting the cell aggregate to be divided, and are integrally connected so as to form a network. With this constitution, the problem of the conventional mesh is suppressed, and a large-grown cell aggregate can be preferably divided.

### (Size limitation on opening shape of through-hole)

The equivalent-circle-diameter of the opening shape of the through-hole varies depending on the type of cells constituting the cell aggregate to be divided. For example, when the cell is a pluripotent stem cell, an embryonic stem cell, or the like, it is about 40 µm - 90 µm, more preferably 50 µm - 80 µm, further preferably 60 µm - 70 µm. In the following, preferable size and the like of each part are illustrated for cases where the cell is a pluripotent stem cell, an embryonic stem cell or the like, but other cells may also be changed to have appropriate sizes.

With only the above-mentioned definition of the aforementioned equivalent-circle-diameter, an elongated opening shape such as a slit and an intricate opening shape such as a maze are also included. Therefore, in the present invention, in addition to the aforementioned limitation on the equivalent-circle-diameter, the opening shape being a shape capable of accommodating a circle having a diameter of 35 µm to 85 µm (hereinafter referred to as contained circle) is added to the limiting condition. Here, the opening shape being able to accommodate the contained circle also includes the case where the contained circle is inscribed in the opening shape and the case where the contained circle matches the opening shape.

When shapes having the same equivalent-circle-diameter are compared, regular hexagon has a larger diameter of the contained circle than a square. In the case of a square corresponding to the preferred lower limit of the equivalent-circle-diameter of 40 µm, the length of one side of such square is about 35.449 µm, in which case the diameter of the contained circle is about 35.449 µm or less. Therefore, in the present invention, 35 µm is set as a preferable lower limit of the diameter of the contained circle. In the case of a regular hexagon corresponding to 90 µm, which is a preferable upper limit of the equivalent-circle-diameter, the distance between two opposing sides in such regular hexagon is about 85.708 µm, and the diameter of the contained circle in this case is about 85.708 µm or less. Therefore, in the present invention, 85 µm is set as a preferable upper limit of the diameter of the contained circle.

The diameter of the aforementioned contained circle is more preferably 44 µm - 76 µm, further preferably 53 µm - 67 µm. When the opening shape is circular, the diameter of the contained circle = equivalent-circle-diameter, otherwise, the diameter of the contained circle <equivalent-circle-diameter.

### (Opening shape of through-hole)

The opening shape of a large number of through-holes provided in the mesh structure is not particularly limited, and may be circular, elliptical, triangular, quadrangular, hexagonal, or other polygonal or irregular shape. When it is a shape with an acute-angled internal angle such as triangle and the like, the opening ratio (the ratio of the total opening area to the area of the mesh structure) cannot be increased from the viewpoint of film strength, and problems of decrease in the collection rate and the like may occur. In the case of a circular shape, since the width of the beam part is not constant and the area where the beam parts are connected to each other is large, the cutting property of the cell aggregate by the beam part is not preferable. In contrast, in the case of a square or a regular hexagon, since the internal angle is not an acute angle and the area where the beam parts are connected to each other is small, the aforementioned problems are preferably suppressed.

To obtain a uniform cell aggregate after cutting, it is preferable that all opening shapes are congruent with each other.

The width of the beam part (distance between the through-holes adjacent to each other) is preferably uniform because the cuttability along the length of the beam part becomes uniform.

From these aspects, the opening shape of the through-hole is preferably quadrangle or hexagon, and square and regular hexagon with sides (beam part) around the opening that are equal to each other are more preferable. A regular hexagon is a preferable shape since it is closer to a circle.

### (Configuration pattern of opening)

When all the opening shapes are congruent regular hexagons, the arrangement pattern of the openings on the film surface is preferably a close-packing shape, in which case the beam parts are in a honeycomb shape connected to each other to form a net as shown in Fig. 1(a). This embodiment is preferable because width W2 of all the beam parts is uniform except for the portion where the terminal portions of the three beam parts are connected to each other.

When all the opening shapes are congruent squares, the arrangement pattern of the openings on the film surface is preferably a square matrix, in which case the beam parts are arranged in an orthogonal lattice mesh connected to each other as shown in Fig. 3(a). This embodiment is also preferable because width W2 of all the beam parts is uniform except for the portion where the terminal portions of the four beam parts are connected to each other. As shown in Fig. 3(b), it may be an arrangement pattern in which the square openings are laterally offset every other row. Since the beam part in the lateral direction is divided into two like part A surrounded by the dashed line, the cuttability is different from that of the orthogonal grid as shown in Fig. 3(a).

As shown in Fig. 1(a), when the openings of the regular hexagon are arranged in a close-packing shape, of the six sides of the regular hexagon, the distance W1 between two parallel sides facing each other may be the diameter of the aforementioned contained circle. It is preferably 38 µm - 85 µm, more preferably 46 µm - 76 µm, further preferably 57 µm - 67 µm. In this case, the width W2 of the beam part is preferably 10 µm - 60 µm, more preferably 20 µm - 40 µm. As shown in Fig. 3(a), when the openings of the square are arranged in a square matrix shape, of the four sides of the square, the distance W11 between two parallel sides facing each other may be the diameter of the aforementioned contained circle. It is preferably 35 µm - 80 µm, more preferably 44 µm - 71 µm, further preferably 53 µm - 62 µm. In this case, the width W21 of the beam part is preferably 10 µm - 60 µm, more preferably 20 µm - 40 µm.

### (Thickness of main body part)

While the thickness of the film-like main body part is not particularly limited, to make the beam part a thin line, it is preferably 10 µm - 60 µm, more preferably 20 µm - 40 µm.

### (Cross-sectional shape of beam part)

In the following, to explain the constituent, one film surface of the film-like main body part is referred to as a first film surface, and the film surface on the opposite side is referred to as a second film surface. When the device is used, the first film surface is the surface used as the inlet side, and the second film surface is the surface used as the outlet side.

The cross-sectional shape of the beam part (the shape of the cross section perpendicular to the longitudinal direction of the beam part) can be rectangular (right-angled quadrilateral) depending on the relationship between the width W2 of the beam part and the thickness tl of the porous film, as shown in Fig. 1(b) or Fig. 1(c). In such a case, there is no difference between the first film surface and the second film surface, and the first film surface may be used as the outlet side.

On the other hand, the rounded shape of the two corners on the inlet side of the above-mentioned rectangle, as shown in Fig. 1(d) or Fig. 1(e), has a higher survival rate of the divided cell aggregate and may be preferable in some cases. It is considered that this is because the damage to the cells is reduced, the individual cells themselves are less likely to be cut, and the cells are more likely to be separated at the interface where the cells adhere to each other, compared to the case where the two corners on the inlet side are sharp right-angled edges.

In the embodiment of Fig. 1(d), only the two corners on the rectangular inlet side have a locally rounded shape, and therefore, a flat plane remains on the inlet side surface of the beam part. On the other hand, in the embodiment of Fig. 1(e), the two corners on the rectangular inlet side is more rounded than the embodiment of Fig. 1(d), and the entire cross-sectional shape is semicircular (more precisely, a shape with circular arc and chord). Therefore, the surface on the inlet side of the beam part is entirely curved as if it were the surface of a cylindrical body. As mentioned above, to reduce the damage to the cell, a larger radius of the roundness of the corner is preferable, and the embodiment of Fig. 1(e) is more preferable than the embodiment of Fig. 1(d). The radius of the roundness also varies depending on the width W2 of the beam part and the thickness t1 of the film-shaped main body part, and is, for example, about 1 µm - 100 µm. The radius of the roundness may be uniform like a circular arc, or may vary from place to place.

### (Material of main body part)

The material of the film-like main body part is not particularly limited, and metal materials such as gold, silver, copper, iron, zinc, platinum, nickel, chrome, palladium and the like, and alloys consisting of any combination of these materials can be mentioned. Preferred alloy is, for example, stainless steel, brass or the like.

### (Production method of the device)

The production method of the device is not particularly limited and a suitable method according to the material such as resin form, punching out, LIGA (Lithographie Galvanoformung Abformung) and the like can be selected. Since the opening shape and the width of the beam part are minute, a production method using LIGA is exemplified. In the production method using LIGA, for example, a metal mold for electrocasting is created by lithography, and an electrochemical reaction is used in the electrocasting tank to form a metal plating layer to be the device on the surface of the metal mold, and the metal plating layer is peeled off from the mold and used as the device.

A shape in which the two corners on the inlet side of the cross-sectional shape of the beam part are rounded as shown in Fig. 1(d), (e) can also be obtained by, for example, changing the shape of the concave portion of the aforementioned metal mold to a shape with rounded inside-corners.

The method of using the device is basically the same as that of a conventionally known mesh. A method of flowing the cell aggregates to be divided together with a liquid such as a culture solution such that the cell aggregates pass through the mesh structure of the film-like main body part in the thickness direction of the main body part can be mentioned.

Where necessary, two or more of the devices arranged in series may be used. For example, two or more of the devices may be arranged in a stack in one holder, or two or more holders containing one of the devices and connected in series may be used. The specifications of the mesh structure of the device when two or more devices are used may be different from each other or may be the same.

### (Holder for preferably using the device)

In the present invention, a holder for preferably using the device is proposed. By setting the device in the holder, a divider that can be preferably inserted in the middle of the flow path (pipe line) of the closed culture system is configured. The holder not only allows the cell aggregate to be divided to preferably pass through the device together with the liquid, but also makes it possible to continuously perform cell culture, cell aggregate division, and subculture of the cell aggregate after division in a closed system.

Fig. 4 is a cross-sectional view showing one embodiment of the structure of the holder. As shown in the Figure, a holder 40 is composed of a holder body 41 having a configuration surface 41s for arranging the device 1A, and a cap part 42 that covers the device 1A arranged on the configuration surface 41s and detachably fixed to the holder main body. In the embodiment of Fig. 4, the device 22 is sandwiched between two pieces of gaskets (sheet-shaped sealing members) 43 and 44, whereby the liquid medium is sealed so as not to leak out of the holder. The inner surface of the cap part 42 is a pressing surface 42s for pressing the device 1A against the holder main body 41.

The holder main body 41 has a first through-hole 41p that opens in the configuration surface 41s, and the cap part 42 has a second through-hole 42p that opens in a pressing surface 42s. As shown in Fig. 4, when the cap part 42 is fixed to the holder main body 41, the center of the opening of the first through-hole 41p and the center of the opening of the second through-hole 42p coincide with each other. Unlike the conventional mesh, since the front and back surfaces of the device is flat, the liquid medium can be easily and preferably air-tightly sandwiched so that the liquid medium may not leak in the transversely direction.

The outer shape of the gasket is preferably equal to or larger than the outer shape of the device. The materials of the gaskets 43 and 44 may be, for example, silicon or the like, which shows preferable sealability without affecting the living body.

In the embodiment of Fig. 4, at the center of each of the two pieces of gasket, a circular through-hole having the same cross-sectional shape as the first through-hole 41p and the second through-hole 42p is provided. In the embodiment of the Figure, the inner diameter of these through-holes is about 1.6 mm and the through-holes are aligned on a line. As a result, the flow of the liquid medium passes through the device in the cross-sectional shape of these through-holes. If the flow changes as it passes through the device, the divided cell aggregate may also be subjected to the shearing force of the flow, and may become smaller. Therefore, the inner diameter of the first through-hole 41p, the second through-hole 42p, and the through-hole of each of the two pieces of gasket is preferably the same. However, the inner diameters of these through-holes may be different from each other as long as they do not cause a turbulent flow that gives an adverse influence.

In the embodiment of Fig. 4, the inner diameter of the conduit of the first through-hole 41p, the second through-hole 42p is constant, and the ratio of the inner diameter (D1) of the conduit to the inner diameter (d1) of the through-hole of the gasket that determines the effective diameter of the flow passing through the device, (D1/d1), is preferably about 0.33 - 3, more preferably 0.5 - 2, further preferably about 1. In a preferred embodiment, D1/d1=1. In consideration of matching the central axis of the flow path, appropriate allowable sizes may be given to the inner diameter of each through-hole, the outer diameter of the gasket, the diameter of the configuration surface 41s, and the like.

In the embodiment of Fig. 4, a male screw 41t is provided on the outer circumference of the body of the holder main body 41, and a female screw 42t is provided on the inner surface of the body of the cap part 42. These screws screw and fix the holder main body 41 into the cap part 42 and the device 1A is sandwiched and pressed to prevent leaking. A knurl, a head portion of a hexagon head bolt, or the like for exerting a force for rotating (or holding) these may be provided on the outer circumference of each body of the holder main body 41 and the cap part 42.

The attachment/detachment structure between the holder main body and the cap part is not limited to the aforementioned screw structure described above, and may be a one-touch coupling structure or a structure in which the cap part is tightened to the holder main body by using bolts and female screws, or the like.

The material of the holder is not particularly limited, and examples thereof include organic polymer materials such as polystyrene, polypropylene, poly(ethylene terephthalate), polycarbonate, acrylic, silicon, polyvinylidene fluoride and the like, and metal materials such as stainless steel and the like. From the viewpoint of molding at a low cost and resistance to autoclaves and gamma rays, polypropylene, polycarbonate, and acrylic are exemplified as preferable materials.

The inner diameters of the conduits 41p and 42p (the cross-sectional shape is circular) of the holder main body 41 and the cap part 42, respectively, are not particularly limited and may be appropriately determined according to the scale and flow rate of the production system. About 0.5 mm - 15 mm is versatile and useful. In the embodiment of Fig. 4, the inner diameter of the conduit is 1.6 mm.

To connect these pipe lines to external pipes, connecting pipes 41c and 42c protrude respectively from the holder body main body and cap part. The outer surface of the body of these pipes may be, for example, in the shape of a hose nipple (also called "bamb fitting joint"), or may be press-fitted into a soft tube or the like (or a soft tube or the like may be press-fitted) to form connection. It may be a structure having connectivity with a known connector such as a female side or a male side of a known one-touch joint (quick coupling), a push-in joint for a resin tube, a tightening joint, or the like.

The outer shape of the holder is not particularly limited. In the embodiment of Fig. 4, the overall outer shape (excluding the pipe portion) of the holder is cylindrical and has a diameter of 20 mm. The total length is about 20 - 25 mm. These numerical values are examples, and may be appropriately determined according to the inner diameter of the conduit, the structure of the connector, and the like.

By using the holder as described above, the loss of cells remaining (trapped) in the device does not occur, turbulent flow is less likely developed as the cell aggregate passes through the device with the liquid, movement along the layer flow allows the cell aggregate to be cut without resistance, damage to cells is reduced, and the survival rate of the cell aggregate after division can be improved.

### (Cell aggregate to be divided)

The type of cells constituting the cell aggregate to be divided by the device is not particularly limited as long as it is a cell that forms a cell aggregate (also referred to as "spheroid") by suspension culture, and any cell can be used. The cells constituting such cell aggregate can be animal or plant-derived cells, and are particularly preferably derived from animal cells. As an animal species from which such cells are derived, mammals such as rat, mouse, rabbit, guinea pig, squirrel, hamster, vole, platypus, dolphin, whale, dog, cat, goat, bovine, horse, sheep, swine, elephant, common marmoset, squirrel monkey, Macaca mulatta, chimpanzee and human and the like are more preferable. The cells constituting the cell aggregate may be those established as cultured cells or primary cells obtained from biological tissues. Further, the cells constituting the cell aggregate may be pluripotent stem cells, which include embryonic stem cells (ES cells), induced pluripotent stem cells (iPS cells), mesenchymal stem cells, neural stem cells and the like. The cells constituting the cell aggregate may be differentiated cells such as hepatocytes, pancreatic islet cells, kidney cells, nerve cells, corneal endothelial cells, chondrocytes, myocardial cells and the like. Furthermore, the cells constituting the cell aggregate may be cells induced to differentiate from umbilical cord blood, bone marrow, fat, and blood-derived tissue stem cells, or tumorigenic cells, cells transformed by genetic engineering techniques, or cells infected with viral vectors. In one embodiment of the present invention, the cell constituting the cell aggregate is preferably a human-derived pluripotent stem cell, especially human iPS cell. In the present specification, the "suspension culture" means culturing cells or cell aggregates (that is, cell clumps having a three-dimensional structure (spherical or cluster of grapes) formed by a large number of cells) under conditions free of adhesion to an incubator.

### (Size of cell aggregate to be divided)

The outer diameter of the cell aggregate before division to which the device is applied is not particularly limited. When the cell is a pluripotent stem cell, an embryonic stem cell, or the like, 50 µm - 300 µm is preferable, 100 µm - 200 µm is more preferable, and 120 µm - 180 µm is further preferable.

For the outer diameter of the cell aggregate, the area of the cell aggregate image obtained by a microscope (including an electron microscope and an optical microscope) is measured, and the diameter of a circle having an area the same as the area (circle-equivalent diameter) can be adopted.

The cell aggregate having the aforementioned outer diameter is divided by the device to have an outer diameter of about 40 µm - 120 µm, more preferably about 50 µm - 90 µm.

The outer diameter (circle-equivalent diameter) of the divided cell aggregate does not always match the circle-equivalent diameter of the opening shape of the through-hole of the device, and may be larger or smaller than the circle-equivalent diameter of the opening shape. For example, a cell aggregate formed into a long columnar shape by passing through a through-hole may have a circle-equivalent diameter larger than the circle-equivalent diameter of the opening shape depending on the observation angle. A small cell aggregate that did not completely fill the through-hole and passed through same while creating a gap with the inner wall of the through-hole (wall surface of the beam part) may have a circle-equivalent diameter smaller than the circle-equivalent diameter of the opening shape.

### (Method for dividing cell aggregate)

The method for dividing a cell aggregate according to the present invention (hereinafter to be also referred to as the method) uses the device according to the present invention, and has a step of dividing a cell aggregate into smaller cell aggregates by passing the cell aggregate to be divided through the mesh structure of the device. By repeating the process of further continuing the divided cell aggregate (subculture) and re-dividing the large-grown cell aggregate by the device, a large amount of the cell aggregate can be efficiently cultured.

When the cell aggregate to be divided is passed through the mesh structure of the device together with the liquid medium, the flow velocity of the liquid medium varies depending on the cell type, the size of the cell aggregate, the viscosity of the liquid medium, and the like. It is generally 10 mm/sec - 500 mm/sec, preferably 50 mm/sec - 150 mm/sec. As the aforementioned flow velocity, the flow velocity at which the liquid (suspension) enters the divider (or the flow velocity out from the divider) can be adopted. The flow velocity can be obtained based on an operation of pushing out or sucking a predetermined amount of solution at a constant speed in a predetermined time using a liquid feed pump such as a syringe and the like. Further, the flow velocity when the liquid passes through the mesh of the mesh structure can be calculated by dividing the amount by the liquid feed pump by the total opening area of the mesh.

### (Culture system repeating culture and dividing of cell aggregate in closed system)

The aforementioned repetition of culture and division of the cell aggregate may be performed in an open system, but in the present invention, a closed culture system using the device is configured, and repetition of culture and division of the cell aggregate without contact with the outside air is proposed.

Fig. 5 schematically shows one example of the configuration of a closed culture system using the device. In the culture system shown in the Figure, a first culture container 50, a holder 40 holding the device 1A, and a second culture container 60 are connected in this order by pipe lines (piping tubes) P1 and P2. The device 1A held in the holder 40 is shown with a thick dotted line. The piping tube P1 is equipped with a pump 70 for sending a fluid in which cell aggregates are dispersed. The position of the pump may be on the piping tube P2. The pump allows the fluid in the first culture container 50 to pass through the device 1A and move to the second culture container 60. With this constitution, a large number of cell aggregates suspension cultured in the liquid medium in the first culture container 50 and grown to a predetermined size pass through the device 1A together with the liquid medium and are divided without being exposed to the outside air, and can be sent to the second culture container 60. The cell aggregates that have been divided and moved to the second culture container 60 may be suspension cultured in the second culture container 60 until they grow to a predetermined size, and returned to the first culture container 50 through the pipe line P3 at the time of division, or when all cell aggregates have moved from the first culture container 50 to the second culture container 60, they may be returned to the first culture container 50 through the pipe line P3 and suspension cultured in the first culture container 50 until they grow to a predetermined size. Alternatively, the cell aggregate that has grown to a predetermined size in the second culture container 60 may be sent to the device 1A together with the liquid medium by reversing the feed direction of the pump 70, passes through the device 1A in the opposite direction of Fig. 5, divided, and returned to the first culture container 50. Alternatively, the cell aggregate that has grown to a predetermined size in the second culture container 60 may pass through another device (net shown) with the liquid medium, divided and sent to a third culture container (not shown). In either embodiment, the cell aggregate grown to a predetermined size is divided by the device, sent to the next culture container, the culture is continued, and divided again by the device. By repeating culture and division automatically or semi-automatically in a closed system in this way, the difference in cell aggregate collection rate due to the difference in skill level of the operator in the procedure can be further reduced, and the cell aggregate can be grown while keeping the culture environment hygienic, that is, while continuing the culture aseptically.

Fig. 6 schematically shows another example of the configuration of a closed culture system using the device. In the culture system shown in the Figure, a first culture container 50, and a holder 40 holding the device 1A are connected by pipe line (piping tube) P1, and a pipeline (piping tube) P4 is set so that the cell aggregate that has passed through the device 1A can be returned to the first culture container 50. The piping tube P1 is equipped with a pump 70 for sending a fluid in which cell aggregates are dispersed. The position of the pump may be on the piping tube P4. The pump allows the fluid in the first culture container 50 to be returned to the first culture container 50 by passing through the device 1A. With this circulation constitution, a large number of cell aggregates suspension cultured in the liquid medium in the first culture container 50 and grown to a predetermined size pass through the device 1A together with the liquid medium and are divided without being exposed to the outside air, sent to the first culture container 50 and mixed with the cell aggregates before division. As a result, both the cell aggregate before division and the cell aggregate after division pass through the device 1A. The cell aggregate after division moves along the flow of fluid and has a high probability of passing through the through-hole of the device 1A without being cut by the beam part of the device 1A. Therefore, it becomes possible to increase the concentration (presence ratio) of cell aggregates in the medium by continuing the circulation of performing suspension culture in the first culture container 50 while dividing a part thereof by the device 1A and returning same to the original state. When the cell aggregate grows to a certain concentration, a part or all of the cell aggregate may be collected.

By repeating culture and division automatically or semi-automatically in a closed system in this way, it is possible to further reduce the difference in cell aggregate collection rate caused by the difference in the skill level of the operator, and cell aggregate can be grown while maintaining the culture environment hygienically, that is, while continuing the culture aseptically.

The system constitution shown in Fig. 5 has an advantage that the size of the cell aggregate can be easily controlled because all the cell aggregates in one culture container are similarly divided and all moved to another culture container; however, it takes time and effort to transfer cell aggregates from a culture container to a culture container. In contrast, in the system constitution shown in Fig. 6, since large and small cell aggregates continue to circulate in the closed loop, the size of the cell aggregate is not uniform; however, it does not take time and effort to transfer cell aggregates from a culture container to a culture container. Therefore, these systems may be used in applications where respective defects do not pose a problem.

In the system shown in Fig. 6, even if cell aggregates of a non-uniform size are mixed, by passing through a mesh or a net-like filter such as the device, it is possible to pass a small cell aggregate and collect only a cell aggregate having a predetermined size or larger by stopping them with the filter. As the net-like filter used for collection, it is preferable to use one that does not cut cell aggregates with ease, such as a mesh made of a thick wire or a mesh structure in which the width of the beam part of the device is made wider. It is preferable to lower the flow velocity than in the case of division so that the cell aggregate can be trapped more preferably.

Fig. 5 and Fig. 6 show examples of circulating a pipe line as a closed loop. A constitution may be adopted in which the cell aggregate and a liquid medium are fed to the device from a source for first supplying the cell aggregate to be divided and the liquid medium (i.e., the liquid medium and the cell aggregate present in the solution thereof), and received by the first container. The source may be an external culture container containing the cell aggregate and the liquid medium, or a container containing the cell aggregate obtained by the systems themselves shown in Fig. 5 and Fig. 6. Furthermore, using plural closed systems shown in Fig. 5 and Fig. 6, and using a container containing the cell aggregate recovered from the first stage system and a liquid medium as a source, a constitution may be adopted in which a required number of closed systems are connected in multiple stages and airtightly, and the cell aggregate and the liquid medium are sequentially supplied to the second and subsequent systems. As a result, the cycle of cell aggregate division and suspension culture can be repeated as many times as necessary, and the cell aggregate can be collected in the required proportion for each cycle.

### (Culture container)

The culture containers (50, 60) shown in the examples of Fig. 5 and Fig. 6 are not particularly limited, and a container capable of accommodating a liquid medium and cells/cell aggregate without affecting the cells can be used, and preferable examples include a relatively hard container, a culture bag made of a flexible film and the like. The culture bag does not require addition of air or the like since the volume of the culture bag can be changed when the liquid medium and cell/cell aggregate are taken out to the outside or when the liquid medium and cell/cell aggregate are put thereinto from the outside. Therefore, a fluid can be preferably moved while maintaining the closed system.

### (Pump, piping)

A pump usable for the systems of Fig. 5 and Fig. 6, is not particularly limited, and peristaltic pumps such as a tube pump (also called roller pump) and syringe pumps are preferable. In particular, a peristaltic pump is preferable since closed piping can be easily constructed. The peristaltic pump is a pump that moves the liquid in the set tube by moving the position at which the elastic and flexible pumping tube is crushed in the feed direction. Even with a peristaltic pump, a large number of cell aggregates are preferably delivered with the liquid medium without being crushed. For more information on the feed structure of the peristaltic pump, prior art can be referred to. When a peristaltic pump is used, the tube for piping preferably has a part that can be attached to the peristaltic pump as a pumping tube and has a shape and flexibility permitting function and operation as a pumping tube.

The connector and coupling for piping are not particularly limited, and it is preferable to use a connector that can be connected aseptically, such as a sterile connector and the like.

### (Liquid medium)

The liquid medium that can be used for the aforementioned cell culture is not particularly limited, and includes a medium suitable for the cells to be cultured and that can form a cell aggregate as a result of culturing the cells in a floating state. Examples of such a medium include a medium capable of sphere culture and a medium containing a specific polysaccharide, and a medium containing a specific polysaccharide is more preferable from the viewpoint of cell culture efficiency and the like (see WO2014/017513 for the detail). Examples of the polysaccharide contained in such a medium include deacylated gellan gum, daiyutan gum, carrageenan and xanthan gum, and salts thereof, and deacylated gellan gum is preferable. By adding such a polysaccharide to a known medium, a liquid medium that can be used for the aforementioned cell culture can be easily prepared. Examples of the known medium that can be used when the cell is derived from an animal include Dulbecco's Modified Eagle's Medium (DMEM), hamF12 medium (Ham's Nutrient Mixture F12), DMEM/F12 medium, McCoy's 5A medium, Eagle MEM medium (Eagle's Minimum Essential Medium; EMEM), αMEM medium (alpha Modified Eagle's Minimum Essential Medium; αMEM), MEM medium (Minimum Essential Medium), RPMI1640 medium, Iscove's Modified Dulbecco's Medium (IMDM), MCDB131 medium, William medium E, IPL41 medium, Fischer's medium, StemPro34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by STEMCELL Technologies), StemSpanSFEM (manufactured by STEMCELL Technologies), StemlineII (manufactured by Sigma Aldrich), QBSF-60 (manufactured by Qualitybiological), StemProhESCSFM (manufactured by Invitrogen), Essential8 (registered trade mark) medium (manufactured by Gibco), Essential8 (registered trade mark) Flex medium (manufactured by Thermo Fisher), StemFlex medium (manufactured by Thermo Fisher), mTeSR (registered trade mark) 1 or 2 or Plus medium (manufactured by STEMCELL Technologies), REPRO FF or REPRO FF2 (manufactured by REPROCELL), PSGro hESC/iPSC medium (manufactured by System Bioscience), NutriStem (registered trade mark) medium (manufactured by Biological Industries), CSTI-7 medium (manufactured by Cell Science & Technology Institute), MesenPRO RS medium (manufactured by Gibco), MF-Medium (registered trade mark) mesenchymal stem cellular proliferation medium (manufactured by TOYOBO), Sf-900II (manufactured by in Invitrogen), Opti-Pro (manufactured by Invitrogen), StemFit (registered trade mark) AK02N or Basic02 or AK03N or Basic03 or Basic04 medium (manufactured by AJINOMOTO HEALTHY SUPPLY), STEMUP medium (manufactured by Nissan Chemical Corporation.) and the like. FCeM (registered trade mark) medium (manufactured by Nissan Chemical Corporation) can be used preferably since it contains polysaccharides that can uniformly disperse cell aggregates.

### (Cell culture, division, collection)

Fig. 7 is a flowchart showing one embodiment of the process of cell culture, division and collection using the device according to the present invention. The movement of the liquid medium (including cell aggregate) between each step is carried out by piping in a closed system without being exposed to the outside air.

As shown in Fig. 7, cell aggregates obtained by detaching colonies grown by adhesion culture from the scaffold (or cell aggregate formed by suspension culture) are divided by passage through the device.

Then, the divided cell aggregates are seeded in a new liquid medium, and suspension cultured to grow the cell aggregates.

Then, the grown cell aggregates are collected, the medium is replaced, cell aggregates are returned to the division step, and divided by passage through the device. At this time, instead of returning all the cell aggregates to the division step, a predetermined ratio of cell aggregates may be taken out as a harvested portion.

### (Preferred embodiment of the method)

The present inventors have found that when the device is continuously used for the division of cell aggregate, solid components such as debris of cell aggregates and fine structures contained in the medium are deposited on the beam part of the mesh structure of the device and, along with the deposition, the effective area of the mesh structure of the device (the total area of openings through which a liquid can pass) gradually decreases, as a result of which the cell aggregate is subject to shear by the high-speed flow and damaged, and divided into small cell aggregates, thereby possibly reducing the survival rate.

Therefore, it is preferable to periodically replace the device with one in which debris of cell aggregates or the like is not deposited.

On the contrary, the present inventors have found that debris of cell aggregates clinging to the beam part of the mesh structure is removed and a decrease in the effective area of the mesh structure is suppressed by flowing a predetermined liquid (such as a liquid medium containing a cell aggregate and a liquid exclusive for washing, which are described later) backward every time a predetermined amount of a liquid containing a cell aggregate passes through the mesh structure of the device. That is, it was found that a decrease in the effective area of the mesh structure can be suppressed by passing a predetermined liquid through the mesh structure in the direction opposite to that at the time of division, as a result of which a decrease in the survival rate of cells contained in the divided cell aggregate can be suppressed. In the following, a predetermined liquid backward flowing process performed to suppress a decrease in the effective area of the net structure is called "backflow washing of the net structure".

### (Backflow washing step to perform backflow washing of mesh structure)

Therefore, in a preferred embodiment of the method, the above-mentioned backflow washing step for performing the backflow washing of the mesh structure described above is further added. The backflow washing step is a step of passing a suspension containing a cell aggregate or a cleaning liquid through the mesh structure in the direction opposite to the direction of passage of the cell aggregate through the mesh structure of the device for division, thereby washing the mesh structure, after a predetermined amount of cell aggregate has been divided in the step of dividing the cell aggregate.

Periodic backflow washing of the mesh structure reduces the number of exchange of the device and thus suppresses the decline in cell survival rate while maintaining a closed system.

### (Predetermined liquid to be flown backward in backflow washing of mesh structure)

The "predetermined liquid" to be flown backward in backflow washing of the mesh structure is not particularly limited, and a liquid that enables the mesh structure to be used continuously can be mentioned. For example, a liquid immediately after passing through the mesh structure (i.e., liquid medium (suspension) containing divided cell aggregates), a liquid medium (not including cell aggregate) similar to the liquid medium used when dividing the cell aggregate, a liquid from which the cell aggregate and fine structures for suspending cells have been removed from the liquid medium used when dividing the cell aggregate, and the like can be mentioned.

### (Embodiment example of backflow washing of mesh structure)

The embodiment of backflow washing of the mesh structure is not particularly limited and includes the following.
(1) An embodiment in which a liquid medium containing the cell aggregate to be divided passes through the device and then the flow is reversed, whereby the liquid medium containing the divided cell aggregates passes through the device in the backward direction.
   (ii) An embodiment in which a liquid medium containing the cell aggregates to be divided passes through the device, the flow path is switched, a liquid (liquid medium, etc.) not containing a cell aggregate or the like is supplied to the downstream side (outlet side) of the device, and the flow is reversed, whereby the liquid medium not containing a cell aggregate or the like passes through the device in the backward direction.
   (iii) An embodiment in which a liquid medium containing the cell aggregates to be divided passes through the device, immediately thereafter a liquid medium not containing a cell aggregate or the like is flown in the same direction, and the flow is reversed after the liquid medium has passed through the device, whereby the liquid medium, not containing a cell aggregate or the like passes through the device in the backward direction.

The frequency of backflow washing of the mesh structure is not particularly limited, and may be every one division, or every two or more divisions, and can be appropriately determined according to the total number of cell aggregates that have passed through a unit area of the mesh structure, or by comprehensively considering the benefit of suppressing a decrease in the survival rate of cells contained in the divided cell aggregate and the disadvantage of labor of backflow washing of the mesh structure and the expansion of the system. Preliminary experiments can determine how many cell aggregates that pass through a unit area of the mesh structure reduce how much the cutting performance of the mesh structure.

The flow velocity and cleaning time of the aforementioned predetermined liquid when performing backflow washing of the mesh structure can be appropriately determined according to the kind of the liquid and the effect of the backflow washing. The flow velocity is not particularly limited and is, for example, about 10 mm/sec - 500 mm/sec, particularly preferably about 50 mm/sec - 300 mm/sec. As the flow velocity, the flow velocity at which the liquid (suspension) enters the divider (or exits the divider) can be adopted. The flow velocity can be obtained based on the operation of extruding or sucking a predetermined amount of solution at a constant speed in a predetermined time using a liquid feed pump such as a syringe and the like. In addition, the flow velocity when the liquid passes through the mesh of the mesh structure can be calculated by dividing the flow rate by the aforementioned liquid feed pump by the total opening area of the mesh. The washing time (backflow time) is also not particularly limited, and when the flow velocity of the liquid is within the aforementioned range, it is about 0.1 sec - 5 sec, particularly preferably, about 0.3 sec - 2 sec.

To perform backflow washing of the mesh structure, a backward direction feed function or backward direction feed apparatus for moving the above-mentioned predetermined liquid may be further provided. The aforementioned backward direction feed function may utilize the backflow function of the liquid feed apparatus provided in the cell culture system using the device, or the backflow function may be further added to the liquid feed apparatus. The backflow function of the liquid feed apparatus may be, for example, reverse rotation of peristaltic pump, reverse operation of syringe pump (suction against extrusion), pressing of a flexible container, and the like. In addition, the backward direction feed apparatus to perform backflow washing of the mesh structure and the piping configuration thereof are not particularly limited. For example, in the constitution of the culture system in Fig. 5, a configuration in which the flow of a liquid medium in the direction of an arrow is simply reversed can be recited. Furthermore, a liquid medium supply source (not shown) is connected to the pipe line P2 via a switching valve (not shown), and the switching valve is switched to supply the liquid such that the liquid medium from the liquid source flows backward through the device 1A. By these operations, a suspension containing a cell aggregate or a new liquid medium passes through the device 1A in the opposite direction, and the cell aggregate and the like entangled with the beam part of the device are removed from the beam part.

### [Example]

### [Experimental Example 1] Division of cell aggregate of human pluripotent stem cells (hiPS cells)

The hiPS cells were suspension cultured to form cell aggregates, the cell aggregates were divided using the device of the present invention and a conventional mesh, and a test was conducted to confirm the division performance of the device of the present invention by observing the survival rate of the cell aggregates after each division.

### (Three-dimensional culture of hiPS cell before division)

### medium 1:

Liquid medium composition prepared by injecting 0.016% (w/v) deacylated gellan gum (KELCOGEL CG-LA, manufactured by Saneigen FFI) using FCeM-series Preparation Kit (manufactured by Nissan Chemical Corporation) to mTeSR1 medium (manufactured by Stem Cell Technologies) containing 10 µM Y-27632 (manufactured by FUJIFILM Wako Pure Chemical Corporation) according to the mixing method described in patent document 2.

### medium 2:

Liquid medium composition prepared by injecting 0.016% (w/v) deacylated gellan gum to mTeSR1 medium according to the mixing method described in patent document 2.

The hiPS cell line 253G1 (distributed from RIKEN) was cultured in a CO₂ incubator (37°C, 5% CO₂) in a static state using a 15 mL tube, medium 1 and medium 2 (pre-division culture).

On day 0 of pre-division culture, hiPS cell line 253G1 was seeded in medium 1, medium 2 was added every 1 to 2 days, and this was continued for 5 - 6 days to form cell aggregates. On the final day, the cell aggregates were centrifuged (100×G, 3 min), the supernatant was removed, and the cell aggregates were suspended in medium 1 and then passed through the device of the present invention to divide the cell aggregates. They were seeded in medium 1 (day 0 of culture after division).

### (Specifications of the device of the present invention)

The instruments used in the following cases were all sterilized.

As an example product of the device of the present invention, a porous film of the type shown in Fig. 1 was produced.

The material of the film body is nickel.

As shown in Table 1 below, the thickness of the film body is 20 µm or 40 µm.

The shape of the opening of each through-hole is a regular hexagon congruent with each other, and the through-hole is arranged on the entire film surface of the film body. The pore size of each through-hole (the distance between two parallel sides facing each other among the six sides of the regular hexagon which is the shape of the opening) is 60 µm or 70 µm as shown in Table 1 below.

The wire diameter (width of the beam part) is 20 µm or 40 µm.

The shape of the outer circumference of the film body is circular, and the size (diameter) of the circle is 13 mm.

### (Holder for holding the device of the present invention)

As shown in Fig. 4, a holder for setting the device was produced. The inner diameter of the conduit (circular cross section) for flowing the liquid medium composition in which cell aggregates are dispersed is 1.6 mm, and the effective diameter of the flow of the liquid medium composition passing through the device is also 1.6 mm.

### (Comparative example: division using mesh)

In Comparative Example, a conventional mesh was used as a device for division.

The tip discharge part (effective diameter of the discharge opening: 1.6 mm) of a 5 mL syringe was covered with a nylon mesh (mesh made of nylon wires) or a stainless mesh (mesh made of stainless steel wires) and fixed with a band.

The opening shape of the through-hole of the nylon mesh is approximately square, and the length of one side is 70 µm. The diameter of the wire is 50 µm for both the warp and weft wires.

The opening shape of the through-hole of the stainless mesh is approximately square, and the length of one side is 70 µm. The diameter of the wire is 40 µm for both the warp and weft wires.

### (Division of cell aggregate of hiPS cell)

After 5 - 6 days of pre-division suspension culture, cell aggregates were precipitated by centrifugation (100×G, 3 min). The supernatant was removed, and the aggregates were suspended in medium 1 to 2.0×10⁵ cells/mL. 4 mL of the suspension was transferred to a 5 mL syringe (manufactured by Terumo Corporation), and the suspension was passed through an example product of the device of the present invention and the mesh of Comparative Example at a predetermined passage speed.

### (Culture after division)

The suspension after passing through the device of the present invention and the mesh of Comparative Example was seeded in a 15 mL tube and cultured in a CO₂ incubator (37°C, 5% CO₂) in a static state (culture after division). The cap of the 15 mL tube was half-opened. On day 2 of culture after division, 2.5 mL each of medium 2 preheated to 37°C was added. On day 4 of culture after division, 3.5 mL each of medium 2 preheated to 37°C was added.

### (Cell survival rate)

Two hrs after the division, the culture tube was removed from the incubator, and the cell aggregates were well dispersed. 0.25 mL of the culture medium was collected and 0.25 mL of ATP reagent (CellTiter-Glo (registered trade mark) Luminescent Cell Viability Assay, manufactured by Promega) was added and the mixture was stirred with Pipetman. After allowing to stand at room temperature for 10 min, 100 µL of each was dispensed into a white 96-well plate, the luminescence intensity (RLU value) was measured with Enspire (manufactured by Perkin Elmer), and the number of viable cells was measured by subtracting the luminescence value of the medium alone.

The relative value when the RLU value (ATP measurement, luminescence intensity) of the suspension before division was 100% was taken as the cell viability.

### (Cell proliferation rate)

On day 5 of culture after division, the culture tube was removed from the incubator, and the cell aggregates were well dispersed. 0.5 mL of the culture medium was collected and 0.5 mL of ATP reagent (CellTiter-Glo (registered trade mark) Luminescent Cell Viability Assay, manufactured by Promega) was added and the mixture was stirred with Pipetman. After allowing to stand at room temperature for 10 min, 100 µL of each was dispensed into a white 96-well plate, the luminescence intensity (RLU value) was measured with Enspire (manufactured by Perkin Elmer), and the number of viable cells was measured by subtracting the luminescence value of the medium alone. Taking into consideration the culture volume ratio, a relative value with the RLU value (ATP measurement, luminescence intensity) of the culture medium 2 hrs after the division was taken as the cell proliferation rate.

### (Size and number of cell aggregates after division)

Two hrs after the division, the culture tube was removed from the incubator, and the cell aggregates were well dispersed. 1 mL of the culture medium was transferred to a 6-well plate, and the size and number of the cell aggregates were measured with cell³iMager (manufactured by SCREEN Holdings) . The culture medium used for the measurement was not returned to the tube. From the measurement results, the circle-equivalent diameter, the number of cell aggregates, and the proportion of cell aggregates having a circle-equivalent diameter of 120 µm or more were calculated.

The above test results are shown in the following Table 1.

**[Table 1]**

| Case | form of mesh | pore size (µm) | wire diameter (µm) | passage speed (cm/sec) | cell viability (%) | cell proliferation rate | circle-equivalent diameter (µm) | proportion of cell number aggregates with diameter of 120 µm or more | |
|---|---|---|---|---|---|---|---|---|---|
| (before crushing) | - | - | - | - | 100 | - | 149.0 | 214 | - |
| comparison example 1 | nylon mesh | 70 | 50 | 15 | 97 | 3.73 | 95.9 | 535 | 22.1% |
| comparison example 2 | stainless mesh | 70 | 40 | 15 | 87 | 4.13 | 92.9 | 542 | 17.0% |
| comparison example 3 | nylon mesh | 70 | 50 | 7.5 | 74 | 3.82 | 105.1 | 260 | 28.8% |
| comparison example 4 | stainless mesh | 70 | 40 | 7.5 | 76 | 3.23 | 96.7 | 322 | 19.6% |
| Example 1 | porous film | 70 | 40 | 15 | 75 | 3.78 | 88.4 | 522 | 14.4% |
| Example 2 | porous film | 70 | 20 | 15 | 71 | 4.01 | 90.1 | 520 | 14.7% |
| Example 3 | porous film | 60 | 20 | 15 | 77 | 3.98 | 80.4 | 654 | 6.9% |
| Example 4 | porous film | 70 | 20 | 7.5 | 86 | 4.49 | 91.4 | 461 | 14.1% |
| Example 5 | porous film | 60 | 20 | 7.5 | 76 | 3.66 | 83.6 | 605 | 7.6% |

As is clear from Table 1 above, it was clarified that, in the processing speed region of not more than 15 cm/sec, the example product of the device of the present invention could divide into smaller cell aggregates and the number of cell aggregates after the division was larger than those of the woven mesh of Comparative Example.

Also, it was clarified that the device of the Example can reduce the proportion of cell aggregates of 120 µm or more and can divide into cell aggregates with more uniform size than the mesh of Comparative Example.

In the above-mentioned test, in both Comparative Example and Example, it was clarified that a higher cell viability (collection rate) than before can be achieved by reducing the amount of trapping by decreasing the effective diameter of the opening of the through-hole of the device for division and the mesh according to the processing amount, and, in the Example, using a holder with less loss during the division.

### [Experimental Example 2] Confirmation of the effect of backflow washing of mesh structure

In this Experimental Example, the effect of backflow washing on the mesh structure was investigated by repeating the following operations (i) - (iii).
(i) A sample solution containing cell aggregates having a predetermined density is used, and the cell aggregate is divided by the mesh structure of the device at a predetermined flow velocity.
(ii) Every time a predetermined amount of cell aggregates passes, the survival rate of the cell aggregates that have passed through is measured.
(iii) Every time a predetermined amount of the cell aggregates has passed through in the aforementioned (ii), backflow washing is performed on the mesh structure.

The cell aggregate used in the test is a cell aggregate composed of human pluripotent stem cells (hiPS cells).

### (Three-dimensional culture of hiPS cell before division)

### medium 1:

Liquid medium composition prepared by injecting 0.016% (w/v) deacylated gellan gum (KELCOGEL CG-LA, manufactured by Saneigen FFI) using FCeM-series Preparation Kit (manufactured by Nissan Chemical Corporation) to mTeSR1 medium (manufactured by STEMCELL Technologies) containing 10 µM Y-27632 (manufactured by FUJIFILM Wako Pure Chemical Corporation) according to the mixing method described in patent document 2.

### medium 2:

Liquid medium composition prepared by injecting 0.016% (w/v) deacylated gellan gum to mTeSR1 medium according to the mixing method described in patent document 2.

The hiPS cell line 253G1 (distributed from RIKEN) was maintenance cultured in a CO₂ incubator (37°C, 5% CO₂) in a static state using a variable volume 200 mL culture bag (manufactured by Nipro), medium 1 and medium 2.

The cells were seeded in medium 1 on day 0 of culture, medium 2 was added every 1 to 3 days, and this was continued for 6 to 8 days to form a cell aggregate.

On the final day, the cell aggregates were collected using MACS (registered trade mark) Smart Stratiners (70 µm, manufactured by MACS), suspended in medium 1, and then passed through a device according to the device of the present invention, and the divided cell aggregates were seeded (day 0).

This was repeated to carry out maintenance culture of the cells.

### (Specifications of the device)

The instruments of each part used in this test were electron beam sterilized.

As an example product of the device, a porous film of the type shown in Fig. 1 was produced.

The material of the porous film is nickel, the thickness of the porous film is 20 µm, and the width of the beam part is 20 µm. The pore size of each through-hole (the distance between two parallel sides facing each other among the six sides of the regular hexagon which is the shape of the opening) is 70 µm.

The matters except for those specified are the same as in the porous film used in Experimental Example 1.

The shape of the outer circumference of the porous film is circular, and the diameter of the circle is 6 mm.

### (Holder for holding mesh structure)

As shown in Fig. 4, a holder for setting the mesh structure was produced. A preferable divider can be provided by setting the device in the holder. The inner diameter of the conduit (circular cross section) for flowing the liquid medium composition in which cell aggregates are dispersed is 2.6 mm, and the effective diameter of the flow of the liquid medium composition passing through the device is also 2.6 mm.

### (Division of cell aggregate of hiPS cell)

After suspension culture for 7 days, the cell aggregates were collected using MACS (registered trade mark) Smart Stratiners (70 µm, manufactured by MACS), and suspended in medium 1 to produce two kinds of suspensions having different concentrations (3.0×10⁵ cells/mL and 6.0×10⁵ cells/mL).
(i) Experimental Example in which division was continued without backflow washing of the mesh structure.
   50 mL each of the aforementioned two kinds of suspensions was transferred to two 50 mL syringes (manufactured by Nipro), each suspension was passed through the device at a rate of 10 cm/sec, dispensed into a 15 mL tube each time 10 mL of the suspension passed through the device, and 5 tubes containing the sample after division of the suspension at a concentration of 3.0×10⁵ cells/mL and 5 tubes containing the sample after division of the suspension at a concentration of 6.0×10⁵ cells/mL were obtained.
(ii) Experimental Example in which division was continued while performing periodic backflow washing of mesh structure

An operation was repeated in which 50 mL each of the aforementioned two kinds of suspensions was transferred to two 50 mL syringes (manufactured by Nipro), each suspension was passed through the device at a rate of 10 cm/sec, dispensed into a 15 mL tube each time 10 mL of the suspension passed through the device, and the syringe was operated to allow 1 mL of the suspension to flow backward to perform backflow washing of the mesh structure after distribution to the tube, after which 10 mL suspension was re-flown, divided, and distributed to another 15 mL tube. As a result, similar to the aforementioned (i), 5 tubes containing the sample after division of the suspension at a concentration of 3.0×10⁵ cells/mL and 5 tubes containing the sample after division of the suspension at a concentration of 6.0×10⁵ cells/mL were obtained.

The 15 mL tubes (4 kinds, 20 tubes in total) after distribution were allowed to stand in an incubator (37°C, 5% CO₂) for 2 hrs.

### (Measurement of cell survival rate)

After standing for 2 hrs, the aforementioned 15 mL tube was removed from the incubator, the cell aggregates were well dispersed by blending with inversion. 0.75 mL of the culture medium was collected and 0.75 mL of ATP reagent (CellTiter-Glo (registered trade mark) Luminescent Cell Viability Assay, manufactured by Promega) was added and the mixture was well stirred with Pipetman. After allowing to stand at room temperature for 10 min, 100 µL of each was dispensed into a white 96-well plate, the luminescence intensity (RLU value) was measured with Enspire (manufactured by Perkin Elmer), and the number of viable cells was measured by subtracting the luminescence value of the medium alone.

The relative value when the RLU value (ATP measurement, luminescence intensity) of the suspension before division was 100% was taken as the cell viability.

### (Evaluation of effect of backflow washing of mesh structure)

The graphs of Figs. 8(a) and (b) show the results of Experimental Example in which the division was continued without performing the backflow washing of the mesh structure of the above-mentioned (i). The graph of Fig. 8(a) shows the results relating to a suspension having a cell concentration of 3.0×10⁵ cells/mL, and the graph of Fig. 8(b) shows the results relating to a suspension having a cell concentration of 6.0×10⁵ cells/mL.

The graphs of Figs. 8(c) and (d) show the results of Experimental Example in which the division was continued while performing the backflow washing of the mesh structure of the above-mentioned (i). The graph of Fig. 8(c) shows the results relating to a suspension having a cell concentration of 3.0×10⁵ cells/mL, and the graph of Fig. 8(d) shows the results relating to a suspension having a cell concentration of 6.0×10⁵ cells/mL.

As shown in the graphs of Fig. 8(a) and (b), when the backflow washing of the mesh structure was not performed, cell viability decreased as the amount of the dividing treatment increased at both cell densities. In contrast, as shown in the graphs of Fig. 8(c) and (d), when the backflow washing of the mesh structure was performed, a decrease in the cell survival rate was sufficiently suppressed at a cell density of 3×10⁵ cells/mL, and a decrease in the cell survival rate was suppressed even at a cell density of 6×10⁵ cells/mL as compared to the results shown in the graph of Fig. 8(b).

From the above, it was clarified that in the division of the cell aggregate using the mesh structure, the periodic backflow washing of the mesh structure is very effective in suppressing a decrease in the survival rate of the cell aggregate after the division. In addition, it is considered that the decrease in cell survival rate can be suppressed by increasing the frequency of backflow washing of the mesh structure (i.e., at time point when a predetermined number of cell aggregates have passed through a unit area of the mesh structure) since more cell aggregates pass through the mesh structure when the cell density is higher.

### [Experimental Example 3] Test on the relationship between the cross-sectional shape of the beam part and the survival rate of the cell aggregate

Human pluripotent stem cells (hiPS cells) were suspension cultured to form cell aggregates, and the cell aggregates were divided by four types of devices having different cross-sectional shapes of the beam part, and a test was conducted to confirm the division performance by the cross-sectional shape of the beam part by observing the survival rate of the cell aggregate after each division for each volume.

### (Three-dimensional culture of hiPS cell before division)

### medium 1:

Liquid medium composition prepared by injecting 0.016% (w/v) deacylated gellan gum (KELCOGEL CG-LA, manufactured by Saneigen FFI) using FCeM-series Preparation Kit (manufactured by Nissan Chemical Corporation) to mTeSR1 medium (manufactured by STEMCELL Technologies) containing 10 µM Y-27632 (manufactured by FUJIFILM Wako Pure Chemical Corporation) according to the mixing method described in patent document 2.

### medium 2:

Liquid medium composition prepared by injecting 0.016% (w/v) deacylated gellan gum to mTeSR1 medium according to the mixing method described in patent document 2.

The hiPS cell line 253G1 (distributed from RIKEN) was maintenance cultured in a CO₂ incubator (37°C, 5% CO₂) in a static state using a variable volume 200 mL culture bag (manufactured by Nipro), medium 1 and medium 2.

The cells were seeded in medium 1 on day 0 of culture, medium 2 was added every 1 to 3 days, and this was continued for 6 to 8 days to form a cell aggregate. On the final day, the cell aggregate was collected using MACS (registered trade mark) Smart Stratiners (70 µm, manufactured by MACS), suspended in medium 1, and then passed through a device according to the device of the present invention, and the divided cell aggregates were seeded (day 0). This was repeated to carry out maintenance culture of the cells.

### (Specifications of the mesh structure of the device)

The instruments used in the following cases were sterilized with ethanol for disinfection.

As an example product of the device, a porous film having the following shape was produced.
(a) The shape of the opening is the regular hexagon shown in Fig. 1(a), and the cross-sectional shape of the beam part is the rectangle shown in Fig. 1(c).
(b) The shape of the opening is the square shown in Fig. 3(a), and the cross-sectional shape of the beam part is the rectangle shown in Fig. 1(c).
(c) The shape of the opening is the regular hexagon shown in Fig. 1(a), and the cross-sectional shape of the beam part is the shape (having a circular arc and a chord) with rounded corners on the inlet side shown in Fig. 3(e)).
(d) The shape of the opening is the square shown in Fig. 3(a), and the cross-sectional shape of the beam part is the shape (having a circular arc and a chord) with rounded corners on the inlet side shown in Fig. 3(e)).

The material of the film body is nickel, the thickness of the film body (thickness t1 in Fig. 1(c), (e)) is 20 µm, and the wire diameter (width of the beam part) is 50 µm. The pore size of each through-hole (the distance between two parallel sides facing each other among the six sides of the regular hexagon which is the shape of the opening or the distance between the two sides of a square, which is the shape of the opening) is 60 µm.

The shape of the outer circumference of the film body is circular, and the size (diameter) of the circle is 6 mm.

### (Holder for holding the device of the present invention)

As shown in Fig. 4, a holder for setting the mesh structure was produced. The inner diameter of the conduit (circular cross section) for flowing the liquid medium composition in which cell aggregates are dispersed is 3.0 mm, and the effective diameter of the flow of the liquid medium composition passing through the mesh structure is also 3.0 mm.

### (Division of cell aggregate of hiPS cell)

After suspension culture for 7 days, the cell aggregates were collected using MACS (registered trade mark) Smart Stratiners (70 µm, manufactured by MACS), suspended in medium 1 to a cell density of 3.0×10⁵ cells/mL. 45 mL of each suspension was transferred to a 50 mL syringe (manufactured by Nipro), the suspension was passed through an example product of the device of the present invention at a processing speed of 10 cm/sec, and dispensed into a 15 mL tube every 15 mL.

In addition, division including a backflow washing step was also performed by returning syringe with 1 mL every 10 mL.

The 15 mL tube after dispensing was allowed to stand in an incubator (37°C, 5% CO₂).

### (Cell survival rate)

After 2 hrs from the division, the dispensed 15 mL tube was removed from the incubator, the cell aggregates were well dispersed by blending with inversion. 0.75 mL of the culture medium was collected and 0.75 mL of ATP reagent (CellTiter-Glo (registered trade mark) Luminescent Cell Viability Assay, manufactured by Promega) was added and the mixture was well stirred with Pipetman. After allowing to stand at room temperature for 10 min, 100 µL of each was dispensed into a white 96-well plate, the luminescence intensity (RLU value) was measured with Enspire (manufactured by Perkin Elmer), and the number of viable cells was measured by subtracting the luminescence value of the medium alone.

The relative value when the RLU value (ATP measurement, luminescence intensity) of the suspension before division was 100% was taken as the cell survival rate.

The results of the above are shown in the graph of Fig. 9.

As is clear from the results shown in the graph of Fig. 9, it was found that the cell survival rate decreases in a dose-dependent manner under all conditions. Furthermore, it was found that the cell survival rate is higher when the opening shape is square than when it is a regular hexagon, and that the cell survival rate is higher when the cross-sectional shape of the beam part is rounded at the corner on the inlet side than when it is rectangular.

The results regarding the shape of the opening in this test are not simply influenced by the shape of the opening. Since the opening area of the regular hexagon is 3118 µm² and the opening area of the square is 3600 µm², it is also considered that higher cell survival rate is achieved with the square having a larger opening area.

From the above, it was clarified that it is very effective, in the division when scaled up, to make the cross-sectional shape of the beam part a rectangular shape with rounded corners on the inlet side.

### [Industrial Applicability]

According to the device and the method of the present invention, the problems of the conventional mesh can be resolved, cell aggregates can be more preferably divided, and culture and division in a closing system is made possible.

This application is based on a patent application No. 2018-148033 filed in Japan (filing date: August 6, 2018), the contents of which are incorporated in full herein.

### [Explanation of Symbols]

1 film-like main body part 1
10 mesh structure
20 through-hole
30 beam part

## Claims

1. A device for dividing a cell aggregate into smaller cell aggregates, the device comprising a film-like main body part, wherein
a predetermined region on a film surface of the main body part has a mesh structure with many through-holes disposed on the film surface, the mesh structure comprises many through-holes penetrating the predetermined region in the film thickness direction, and a beam part serving as a partition between the through-holes,
the through-holes have an opening shape of a size permitting passage of the smaller cell aggregates, and
the beam part is a remainder after subtracting the through-hole from the main body part in the predetermined region, is a part that cuts the cell aggregates to be divided, and is integrally connected to form a network.

2. The device according to claim 1, wherein the opening shape of the through-hole has an opening area with an equivalent-circle-diameter of 40 µm - 90 µm, and a shape accommodating a circle with a diameter of 35 µm - 85 µm.

3. The device according to claim 1 or 2, wherein the beam part has a width of 10 µm - 60 µm that is a separation distance between adjacent through-holes.

4. The device according to any one of claims 1 to 3, wherein said many through-holes have opening shapes of quadrangles congruent with each other, and said beam parts are connected to each other in an orthogonal lattice pattern.

5. The device according to any one of claims 1 to 3, wherein said many through-holes have opening shapes of hexagons congruent with each other, and said beam parts are connected to each other in a honeycomb-shape.

6. The device according to claim 5, wherein the hexagon is a regular hexagon, and, among the six sides of the regular hexagon, a distance between two parallel sides facing each other is 38 µm - 85 µm.

7. The device according to any one of claims 1 to 6, wherein the film surface is a first film surface, a film surface on the opposite side thereof is a second film surface,
when in use of the device, the first film surface is a surface used as an inlet side, the second film surface is a surface used as an outlet side, and
a cross-sectional shape in the perpendicular longitudinal direction of the beam part is a rectangle, or two corners on the inlet side of the rectangle have a round shape.

8. The device according to any one of claims 1 to 7, wherein the cell aggregate to be divided is a cell aggregate composed of pluripotent stem cells.

9. A method for dividing a cell aggregate, comprising a step of dividing a cell aggregate to be divided by passing, using the device according to any one of claims 1 to 8, the cell aggregate together with a liquid through the mesh structure of the device.

10. The method according to claim 9, wherein the flow velocity of the liquid is 10 mm/sec - 500 mm/sec when the cell aggregate to be divided passes through the net-like region in the device together with a liquid.

11. The method according to claim 9 or 10, further comprising a backflow washing step of passing, after division of a predetermined amount of the cell aggregates in the step of dividing the cell aggregate, a predetermined liquid through the mesh structure in the direction, opposite to the direction of passage of the cell aggregate through the mesh structure of the device for division, thereby washing the mesh structure.
